# DEMANDE DE BREVET EUROPEEN

(11) **EP 1 064 989 A1**
(43) Date de publication de la demande: **03.01.2001**
(21) Numéro de dépôt: 00401491.6
(22) Date de dépôt: 26.05.2000
(51) Int. Cl.: B01F 17/00, A61K 7/48, A61K 7/06

(54) **Emulsion comprenant une huile volatile fluorée et un tensioactif siliconé, composition cosmétique comprenant ladite émulsion et utilisation dudit tensioactif pour préparer une émulsion stable**

(30) Priorité: 30.06.1999 FR 9908378
(71) Demandeur: L'OREAL, 75008 Paris (FR)
(72) Inventeur: Lorant, Raluca, 94320 Thiais (FR)
(74) Mandataire: Dodin, Catherine

(57) **Abrégé**

La présente demande concerne une composition se présentant sous la forme d'une émulsion comprenant une phase aqueuse, une phase grasse et au moins un tensioactif, ladite phase grasse comprenant au moins une huile volatile fluorée et ledit tensioactif étant choisi parmi des tensioactifs siliconés particuliers.

L'émulsion ainsi obtenue présente une bonne stabilité.

L'invention a également pour objet une composition cosmétique ou pharmaceutique comprenant, ou constituée par, ladite émulsion.

## Description

La présente invention a trait à une composition notamment cosmétique, susceptible d'être employée pour le soin de la peau, se présentant sous la forme d'une émulsion et comprenant des huiles fluorées volatiles et des tensioactifs siliconés.

Une partie des compositions utilisées en cosmétique, notamment pour le soin ou le maquillage de la peau, se présentent sous la forme d'émulsions huile-dans-eau (H/E) ou eau-dans-huile (E/H). Ces émulsions comprennent généralement une phase aqueuse, une phase grasse et un émulsionnant qui permet de maintenir la composition sous forme stable. La nature de l'émulsionnant est généralement choisie en fonction de l'effet recherché: sens de l'émulsion, nature de la phase grasse, caractéristiques cosmétiques désirées, etc.
Il est ainsi connu d'employer, pour émulsionner les émulsions huile-dans-eau classiques, des savons d'acides gras et notamment le stéarate de triéthanolamine. Toutefois, ces émulsionnants ne permettent pas d'émulsionner toutes les huiles, en particulier les huiles de silicone. Or, les huiles de silicone possèdent certains avantages cosmétiques : elles présentent notamment l'intérêt d'apporter plus de douceur aux compositions lors de leur application sur la peau.
On a alors proposé de réaliser des émulsions à l'aide de tensioactifs siliconés, par exemple de type polydiméthylsiloxane polyoxyéthyléné et/ou polyoxypropyléné. Une fois encore, on a constaté que ces émulsionnants ne permettaient pas de réaliser des émulsions stables à partir de toute huile, en particulier à partir d'huile fluorée.
Or, l'utilisation en cosmétique d'huiles fluorées, et en particulier d'huiles fluorées volatiles, permet d'obtenir des compositions ayant des propriétés très avantageuses, telles qu'une application aisée, un effet de fraîcheur immédiat dès l'application, une douceur remarquable. Mais l'émulsification des huiles fluorées, dans des émulsions notamment H/E, reste très difficile. En effet, on a constaté que les émulsionnants dits classiques ne permettent pas d'obtenir des émulsions stables et contenant des huiles fluorées volatiles. Les phénomènes d'instabilité observés sont notamment les suivants :
- les émulsions moussent, ce qui se traduit par un changement rédhibitoire de la texture au cours du temps. Ceci peut être dû à la modification de la composition de l'émulsion au cours du temps, par volatilisation d'une partie des huiles volatiles;
- les émulsions se déphasent au cours du temps; en effet, on constate qu'elles sont sous forme de dispersions grossières ce qui démontre une mauvaise émulsification des huiles fluorées pouvant aller jusqu'à une séparation des deux phases, ce qui rend le produit inutilisable;
- la viscosité des émulsions va évoluer au cours du temps, d'où instabilité physico-chimique.

La présente invention a pour but de pallier ces inconvénients et de proposer une composition sous forme d'émulsion, qui est stable en présence d'huiles fluorées, tout en ayant de bonnes propriétés cosmétiques.

La demanderesse a découvert de façon surprenante et inattendue qu'il était possible d'atteindre ce but en utilisant un tensioactif siliconé très particulier.
On entend par émulsion stable dans la présente description une émulsion qui ne décante pas après conservation pendant deux mois à 45 °C.

L'invention a donc pour objet une composition se présentant sous la forme d'une émulsion comprenant une phase aqueuse, une phase grasse et au moins un tensioactif, caractérisée par le fait que ladite phase grasse comprend au moins une huile volatile fluorée et que ledit tensioactif est choisi parmi les tensioactifs siliconés comprenant au moins un groupement anionique.
L'invention a également pour objet une composition cosmétique ou pharmaceutique caractérisée par le fait qu'elle comprend, ou est constituée par, une émulsion telle que ci-dessus définie.
Un autre objet de l'invention est l'utilisation d'un tensioactif siliconé comprenant au moins un groupement anionique pour la préparation d'une émulsion stable comprenant une phase aqueuse et une phase grasse qui comprend au moins une huile volatile fluorée.

On a constaté que l'émulsion selon l'invention s'applique et s'étale de façon homogène, sans laisser de sensation de gras. Le film obtenu présente également une texture légère et reste confortable à porter tout au long de la journée.
Sur la peau, les émulsions sont glissantes, fraîches à l'application, filmogènes et confortables à porter. Elles laissent la peau lisse, fraîche et soyeuse au toucher et apportent un effet de bien-être.
En outre, les tensioactifs utilisés selon l'invention permettent d'émulsionner parfaitement les huiles de silicones qui peuvent ainsi améliorer les propriétés cosmétiques (propriétés lubrifiantes, filmogènes, protectrices, résistance à l'eau) de l'émulsion selon l'invention.
L'utilisation de tensioactifs siliconés modifiés par des groupements anioniques permet de résoudre ces problèmes de stabilité.
De plus, ces tensioactifs permettent d'obtenir des textures particulièrement intéressantes, notamment très douces, ayant un aspect brillant, voire même translucide.
Par ailleurs, certains de ces tensioactifs se présentent sous forme liquide, ce qui permet de préparer les émulsions à température ambiante. Ceci permet notamment d'éviter une étape de chauffage, particulièrement peu recommandée si l'on souhaite utiliser des composés volatils, notamment des huiles fluorées volatiles ou des actifs instables à la chaleur.
On peut ainsi obtenir des émulsions bien tolérées par la peau car les constituants de ladite émulsion présentent une très bonne innocuité.

L'émulsion selon l'invention comprend donc au moins un tensioactif siliconé comprenant au moins un groupement anionique. Ledit tensioactif peut donc être un tensioactif anionique ou un tensioactif amphotère.
Selon l'invention, le groupement anionique présent dans le tensioactif siliconé peut être choisi parmi les groupements phosphate, sulfate, sulfonate et/ou carboxylate.
De préférence, on utilise les tensioactifs siliconés comprenant au moins un groupement phosphate ou sulfate.

Parmi les tensioactifs siliconés à groupement phosphate, on peut citer ceux de formules (I) à (IV) suivantes : 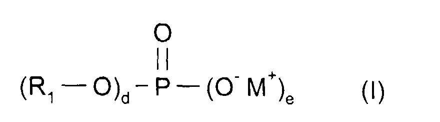 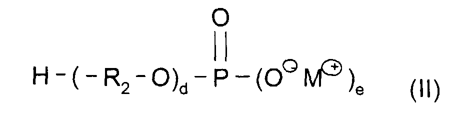 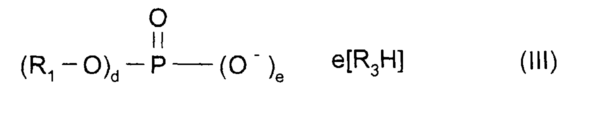 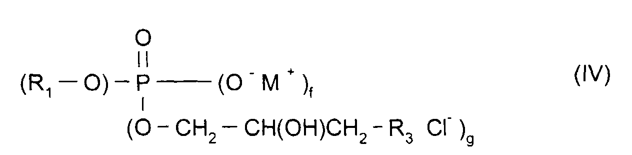 dans lesquelles :
- R₁ désigne le radical de formule (V) suivante : 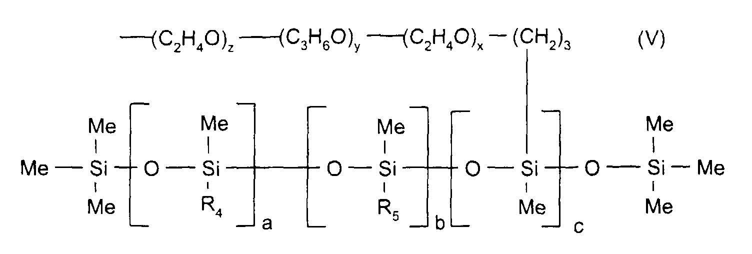
- R₂ est le radical de formule (VI) suivante : 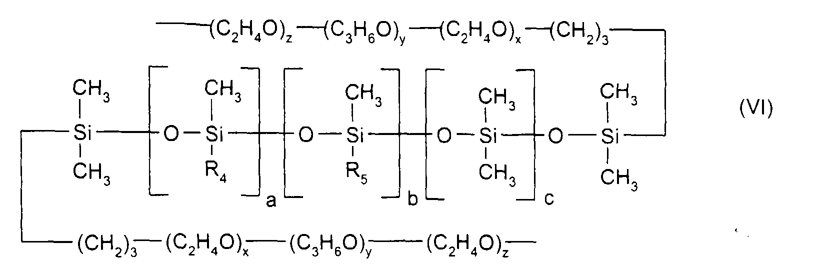 dans lesquelles :
   . Me désigne le radical méthyle,
   . C₂H₄O représente le groupement -CH₂-CH₂-O-,
   . C₃H₆O représente le groupement -CH₂-CH(CH₃)-O-
   . a est un nombre entier allant de 0 à 200,
   . b est un nombre entier allant de 0 à 200,
   . c est un nombre entier allant de 1 à 200,
   . R₄ désigne un radical -(CH₂)ₙCH₃ ou phényle, n étant un nombre entier allant de 0 à 10, et de préférence R₄ désigne un radical méthyle
   . R₅ est un radical -(CH₂)₃-(OCH₂CH₂)ₓ-(OCH₂CH(CH₃))_{y}-(OCH₂CH₂)_{z}-OH, x, y, z sont des nombres entiers, allant indépendamment de 0 à 20, et de préférence x+y+z ≥ 3,
- d et e vont de 1 à 2, avec d+e=3,
- f est égal à 0 ou 1, g est égal à 1 ou 2, avec f+g=2,
- M est choisi dans le groupe constitué de H, Na, K, Li, NH₄ et N(CH₂CH₂OH)₃
- R₃ est choisi parmi : 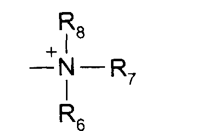 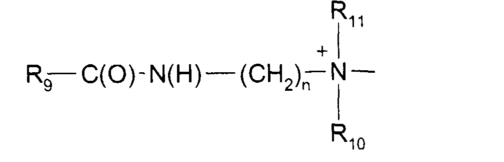 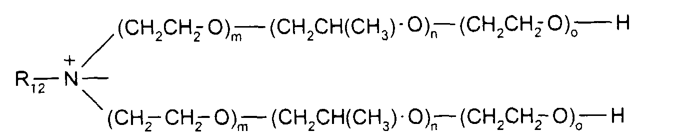 et 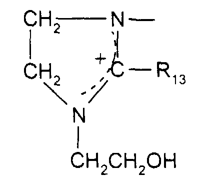 formules dans lesquelles :
   . R₆ à R₉ désignent, indépendamment, un radical alkyle ayant 1 à 20 atomes de carbone,
   . R₁₀ et R₁₁ désignent, indépendamment, un radical alkyle ayant 1 à 3 atomes de carbone,
   . R₁₂ et R₁₃ désignent, indépendamment, un radical alkyle ayant 6 à 20 atomes de carbone,
   . m, n et o désignent, indépendamment un nombre entier allant de 0 à 20.

Les tensioactifs de formule (I) sont notamment décrits dans le brevet US-A-5070171.
Les tensioactifs de formule (II), (III) et (IV) sont notamment décrits respectivement dans les brevets US-A-5149765, US-A-5093452 et US-A-5091493.
Parmi les tensioactifs siliconés à groupement phosphate, on utilise de préférence ceux de formule (I), et en particulier ceux commercialisés sous les dénominations "PECOSIL PS-100", "PECOSIL PS-200" et "PECOSIL WDS-100" par la société PHOENIX CHEMICAL.

Parmi les tensioactifs siliconés à groupement sulfate, on peut citer ceux de formule (VII) suivante : 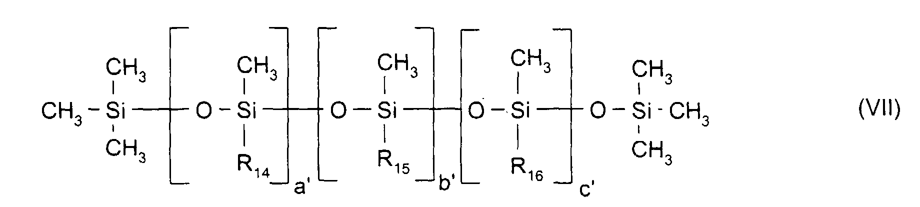 dans laquelle
. R₁₄ désigne un radical alkyle ayant de 1 à 8 atomes de carbone ou un radical phényle,
. R₁₅ désigne un radical
   -(CH₂)₃-O-(CH₂CH₂O)ᵤ-(CH₂CH(CH₃)-O)ᵥ-(CH₂CH₂O)_{w}-SO₃⁻ M⁺,
   M étant choisi parmi Na, K, Li et NH₄,
. R₁₆ désigne un radical
   -(CH₂)₃-O-(CH₂CH₂O)ᵤ-(CH₂CH(CH₃)-O)ᵥ-(CH₂CH₂O)_{w}-H,
   dans lesquels u, v, w désignent, indépendamment, un nombre entier allant de 0 à 100,
. a' et c' désignent, indépendamment, un nombre entier allant de 0 à 50,
. b' désigne un nombre entier allant de 1 à 50, et de préférence c' = 0.
Les composés de formule (VII) sont notamment décrits dans le brevet US-A-4 960 845.

Parmi les tensioactifs siliconés à groupement sulfonate, on peut également citer ceux de formule (VIII) suivante : 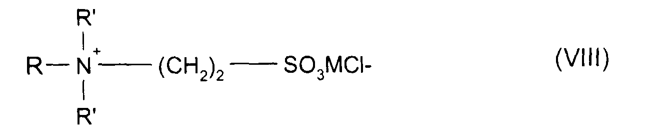 dans laquelle :
. M est choisi dans le groupe constitué de Na, K, Li et NH₄ ,
. R désigne un radical alkyle ayant de 1 à 40 atomes de carbone, et
. R' désigne un radical de formule (V) : 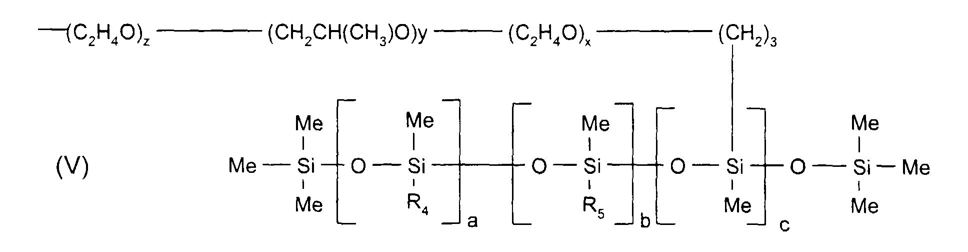 dans laquelle :
. a est un nombre entier allant de 0 à 200,
. b est un nombre entier allant de 0 à 200,
. c est un nombre entier allant de 1 à 200,
. R₄ désigne un radical -(CH₂)ₙCH₃ ou phényle, avec n étant un nombre entier allant de 0 à 10,
. R₅ est un radical -(CH₂)₃-(OCH₂CH₂)ₓ-(OCH₂CH(CH₃))_{y}-(OCH₂CH₂)_{z}-OH, avec x, y et z étant des nombres entiers, allant indépendamment de 0 à 20, et de préférence x+y+z ≥ 3.
De préférence, R désigne un méthyle.

Les composés de formule (VIII) sont notamment décrits dans le brevet US5280099.
Parmi les tensioactifs préférés de formule (VIII), on peut citer le Pecosil DCT de Phoenix (nom CTFA : sodium diméthicone copolyol acetyl méthyltaurate).

Parmi les tensioactifs siliconés à groupement sulfonate, on peut encore citer ceux obtenus par la réaction d'une silicone de formule (IX) : 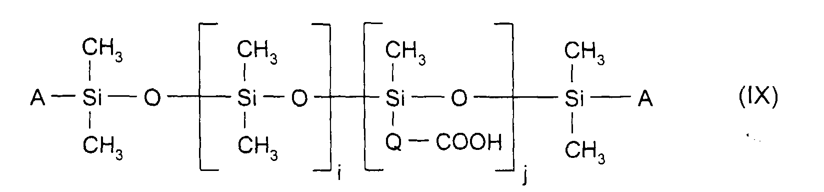 dans laquelle
. Q désigne (CH₂)ᵣ, r étant un nombre entier allant de 3 à 17,
. j désigne soit un nombre entier allant de 1 à 10 et A désigne un radical méthyle, soit j= 0 et A désigne un radical -Q-COOH,
. i désigne un nombre entier allant de 1 à 200,
avec un dérivé de taurine de formule R₁₇-NH-(CH₂)₂-SO₃M, dans laquelle
. R₁₇ désigne un radical alkyle ayant de 1 à 40 atomes de carbone, et
. M est choisi parmi Na, K, Li et NH₄.
Les composés ainsi préparés sont décrits dans le brevet US5286830 dont le contenu est incorporé dans la présente description par référence.

Le tensioactif siliconé utilisé dans le cadre de l'invention est plus préférentiellement choisi parmi les composés de formule (I) et/ou de formule (VIII).

Il peut être présent dans l'émulsion selon l'invention à une teneur allant de 0,5 à 20% en poids, par rapport au poids total de l'émulsion et de préférence de 2 à 7% en poids (en matière active).

L'émulsion comprend par ailleurs une phase aqueuse, qui peut comprendre de l'eau, une eau florale, une eau minérale et/ou une eau thermale.
Ladite phase aqueuse peut être présente à une teneur allant de 50 à 98% en poids par rapport au poids total de l'émulsion, de préférence 70-90% en poids.
En outre, la phase aqueuse peut comprendre 0-14% en poids, par rapport au poids total de la phase aqueuse, d'un monoalcool inférieur en C₂-C₆ et/ou d'un polyol tel que le glycérol, le butylèneglycol, l'isoprèneglycol, le propylèneglycol.

La phase grasse de l'émulsion selon l'invention comprend au moins une huile fluorée volatile. On entend par 'huile volatile', une huile ayant à 25°C, une tension de vapeur saturante au moins égale à 50 Pa.

Parmi les huiles fluorées volatiles susceptibles d'être employées dans le cadre de l'invention, on peut plus particulièrement citer, seul ou en mélange, :
a) les perfluorocycloalkyles répondant à la formule (I) suivante : 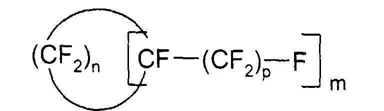 dans laquelle :
   - n est 4 ou 5,
   - m est 1 ou 2, et
   - p est 1, 2 ou 3,
   sous réserve que lorsque m=2, les groupements ne sont pas nécessairement en position alpha l'un par rapport à l'autre ;
b) les perfluoroalcanes répondant à la formule (II) suivante:

   CF₃-(CF₂)ₘ-CF₂X

   dans laquelle :
   - m est 2 à 8, et
   - X représente Br ou F;
c) les fluoroalkyles ou hétérofluoroalkyles répondant à la formule (III) suivante :

   CH₃-(CH₂)ₙ-[Z]ₜ-(CF₂)ₘ-CF₃

   dans laquelle :
   - t est 0 ou 1,
   - n est 0, 1, 2 ou 3,
   - m est 2, 3, 4 ou 5, et
   - Z est O, S ou NR, R étant un hydrogène, un radical -(CH₂)ₘ-CH₃ ou -(CF₂)ₘ-CF_{3,}
d) les dérivés de perfluoromorpholine répondant à la formule (IV) suivante : 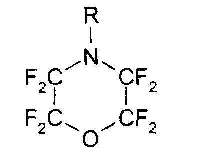 dans laquelle R est un radical perfluoroalkyle en C1-C4.

Parmi les perfluorocycloalkyles de formule (I), on peut notamment citer le perfluorométhylcyclopentane et le perfluoro-1,3 diméthylcyclohexane, notamment vendus sous les dénominations de "Flutec PC1" et "Flutec PC3" par la Société BNFL FLUOROCHEMICALS Ltd., et qui ont des densités respectives égales à 1,26 et 1,82., ou le perfluoro-1,2-diméthylcyclobutane.

Parmi les perfluoroalcanes de formule (II), on peut citer le dodécafluoropentane et le tetradécafluorohexane, notamment vendus sous les dénominations de "PF5050" et "PF 5060" par la Société 3M et qui ont des densités respectives égales à 1,63 et à 1,68, ou encore le bromoperfluorooctyle vendu sous la dénomination de "FORALKYL" par la Société ATOCHEM de densité égale à 1,95.

Parmi les composés fluorés de formule (III), on peut citer le nonafluorométhoxybutane vendu sous la dénomination de "MSX 4518" par la Société 3M, de densité égale à 1,53, ou l'éthoxynonafluoroisobutane.

Parmi les dérivés de perfluoromorpholine, on peut citer la 4-trifluorométhyl perfluoromorpholine vendue par la Société 3M sous la dénomination "PF 5052" de densité égale à 1,7.

Ces huiles fluorées volatiles ont de préférence un indice de réfraction (n_{D}²⁰) inférieur à environ 1,3, un point d'ébullition généralement compris entre 25-65°C et une densité élevée, généralement supérieure à 1, de préférence supérieure à 1,2.

Les huiles fluorées volatiles peuvent être présentes dans l'émulsion en une quantité de 0,5 à 50% en poids par rapport au poids de l'émulsion, de préférence en une quantité de 3 à 20% en poids.

Ces huiles fluorées volatiles peuvent représenter 10 à 100% en poids de ladite phase grasse, de préférence 20 à 80% en poids.

Toutefois, ladite phase grasse de l'émulsion peut comprendre d'autres huiles, fluorées ou non, volatiles ou non, ainsi que tout corps gras usuellement utilisé dans le domaine d'application envisagé.
On peut notamment citer les corps gras siliconés tels que les huiles, les corps gras pâteux, les gommes et les cires de silicone, ainsi que les corps gras non siliconés tels que les huiles, les pâteux et les cires végétales, minérales, animales et/ou synthétiques.
Parmi les corps gras siliconés, on peut citer :
- (i) les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- (ii) les huiles siliconées volatiles, telles que :
   - les silicones volatiles cycliques ayant de 3 à 8 atomes de silicium et de préférence de 4 à 5. Il s'agit par exemple de la cyclotétradiméthylsiloxane, de la cyclopentadiméthylsiloxane ou de la cyclohexadiméthylsiloxane,
   - les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
   - les silicones volatiles linéaires ayant de 2 à 9 atomes de silicium. Il s'agit par exemple de l'hexaméthyldisiloxane, de l'hexyl heptaméthyltrisiloxane ou de l'octyl heptaméthyltrisiloxane,
- (iii) les huiles de silicone phénylées, notamment celles de formule : 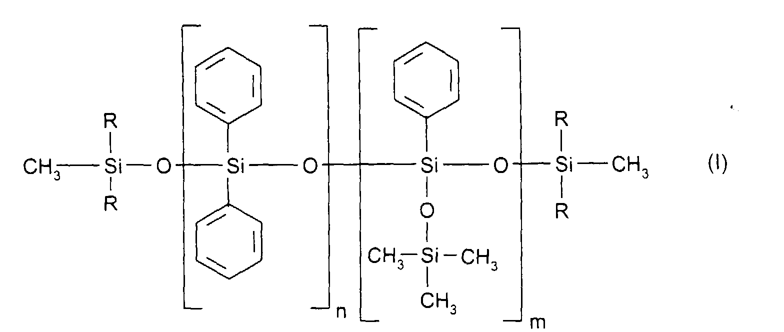 dans laquelle
   . R est un radical alkyle en C1-C30, un radical aryle ou un radical aralkyle,
   . n est un nombre entier compris entre 0 et 100,
   . m est un nombre entier compris entre 0 et 100, sous réserve que la somme est comprise entre 1 et 100.
- (iv) les gommes de silicones et les cires de silicone.

Parmi les corps gras non siliconés, on peut citer :
- (i) les huiles volatiles hydrocarbonées, telles que les isoparaffines et notamment l'isododécane et l'isohexadécane,
- (ii) les huiles et cires usuelles telles que l'huile de paraffine, de vaseline, le perhydrosqualène, l'huile d'abricot, l'huile de germes de blé, d'amande douce, de calophyllum, de palme, de ricin, d'avocat, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides; des huiles hydrogénées concrètes à 25°C; des lanolines; des esters gras concrets à 25°C; la cire d'abeilles; les cires végétales telles que la cire de Carnauba, de Candellila, d'Ouricury, du Japon ou les cires de fibres de lièges ou de canne à sucre; les cires minérales, par exemple de paraffine, de lignite ou les cires microcristallines ou les ozokérites; les cires synthétiques, parmi lesquelles les cires de polyéthylène et les cires obtenues par synthèse de Fischer-Tropsch.

Ces corps gras peuvent en particulier être choisis de manière variée par l'homme du métier afin de préparer une composition ayant les propriétés souhaitées, par exemple en consistance ou en texture.

L'émulsion peut comprendre en outre tout additif usuellement utilisé dans le domaine considéré, tel que des cires, des gommes, des agents épaississants ou gélifiants, des tensioactifs non siliconés, des pigments, des charges, des colorants, des antioxydants, des parfums, des huiles essentielles, des conservateurs, des actifs cosmétiques ou pharmaceutiques, des hydratants, des vitamines, des acides gras essentiels, des sphingolipides, des composés autobronzants tels que la DHA, des filtres solaires, des polymères. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les émulsions selon l'invention peuvent se présenter sous différentes formes et en particulier sous forme d'émulsions huile-dans-eau, eau-dans-huile ou multiples, de consistance liquide ou semi-liquide du type lait, ou de consistance molle, semi-solide ou solide du type crème, gel voire stick.

Ces émulsions peuvent constituer tout ou partie d'une composition cosmétique ou pharmaceutique. Ladite composition peut se présenter sous la forme d'un produit cosmétique, coloré ou non coloré, et notamment sous la forme d'un produit de soin pour le corps, le visage, le cuir chevelu et/ou les cheveux tel qu'une crème, un lait, un gel ou un sérum; d'un produit solaire ou autobronzant; d'un produit de maquillage tel qu'un fond de teint, un fard à joues ou à paupières, un eye-liner, un mascara ou un rouge à lèvres.

L'invention est illustrée plus en détail dans les exemples suivants.

### Exemple 1

On prépare un fluide rafraîchissant et hydratant corporel comprenant :

### phase huileuse

- cyclopentamethicone 5 %
- huile fluorée volatile (MSX 4518 de 3M) 5 %

### phase aqueuse

- glycérol 5%
- conservateurs qs
- gélifiant 0,3%
- tensioactif (Pecosil DCT) 6%
- eau qsp 100%

On disperse la phase huileuse dans la phase aqueuse, à 20°C, sous forte agitation.
On obtient un lait fluide translucide s'étalant facilement et apportant une sensation immédiate de fraîcheur et d'hydratation.

### Exemple 2

On prépare une crème de soin nutritive pour le visage comprenant :

### phase huileuse

- cyclohexamethicone 5 %
- huile fluorée volatile (HFE 7200) 5 %
- huile de noyaux d'abricot 5 %
- vitamines qs

### phase aqueuse

- glycérol 7%
- conservateurs qs
- gélifiant 0,3%
- tensioactif (Pecosil DCT) 6%
- eau qsp 100%

On obtient une crème douce, riche mais non grasse, apaisante et confortable à porter

### Exemple 3

On prépare un lait démaquillant comprenant :

### phase huileuse

- cyclopentamethicone 5 %
- huile fluorée volatile (Flutec PC3 de BNFL Fluorochemicals) 8 %
- adipate de dioctyle 5 %

### phase aqueuse

- glycérol 3 %
- conservateurs qs
- gélifiant 0,3 %
- soude qsp pH 6,5
- tensioactif (Pecosil PS 100) 3%
- eau qsp 100 %

### Exemple 4

On prépare un fond de teint comprenant :

### phase huileuse

- cyclohexamethicone 10 %
- huile fluorée volatile (PF5060 de 3M) 10 %

### phase aqueuse

- glycérol 3%
- conservateurs qs
- gélifiant 0,3%
- tensioactif (Pecosil DCT) 6%
- eau qsp 100 %

### phase poudre

- pigments 7 %
- agents dispersants 0,2 %

On prépare séparément les trois phases. On ajoute la phase poudre dans la phase aqueuse, puis on disperse sous agitation vigoureuse la phase grasse dans la phase aqueuse
On obtient un fond de teint s'étalant facilement sur le visage et qui présente après application une bonne tenue.

### Exemple 5

On réalise plusieurs émulsions H/E en utilisant différents tensioactifs.

### phase huileuse

- tensioactif 4 %
- cyclopentamethicone 5 %
- huile fluorée volatile (MSX 4518 de 3M) 15 %

### phase aqueuse

- glycérol 5%
- conservateurs qs
- gélifiant 0,5%
- eau qsp 100%

On compare les émulsions obtenues et on obtient les résultats donnés dans le tableau ci-dessous.

| Tensioactif (TA) | Observations |
|---|---|
| TA anionique siliconé selon l'invention (Pecosil DCT) | - émulsion très fine et régulière au microscope; - stable 2 mois à 45°C |
| TA anionique non siliconé (stéarate de triéthanol amine) | - émulsion grossière au microscope, non homogène; - mauvaise stabilité: aspect mousseux, hétérogène au cours du temps, à 25°C |
| TA non ionique, non siliconé (glycéryl citrate/lactate) | - émulsion d'aspect hétérogène, nuageuse au microscope; - très instable: mousse énormément |
| TA non ionique, non siliconé (stéarate de PEG + stéarate de sorbitane) | - émulsion d'aspect marbré au microscope; - instable dans le temps: aspect mousseux et épaississement rédhibitoire |

## Revendications

1. Composition se présentant sous la forme d'une émulsion comprenant une phase aqueuse, une phase grasse et au moins un tensioactif, caractérisée par le fait que ladite phase grasse comprend au moins une huile volatile fluorée et que ledit tensioactif est choisi parmi les tensioactifs siliconés comprenant au moins un groupement anionique.

2. Composition selon la revendication 1, dans laquelle le tensioactif siliconé comprend au moins un groupement choisi parmi les groupements phosphate, sulfate, sulfonate et/ou carboxylate.

3. Composition selon l'une des revendications précédentes, dans laquelle le tensioactif siliconé est choisi parmi les composés de formules (I) à (IV) suivantes : 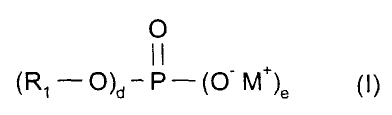 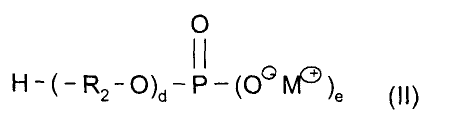 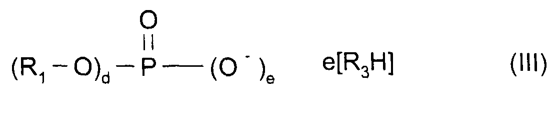 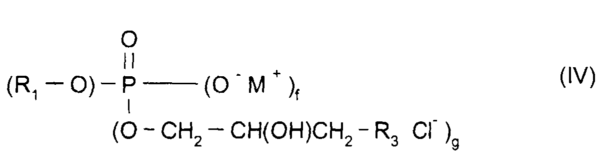 dans lesquelles :
- R₁ désigne le radical de formule (V) suivante : 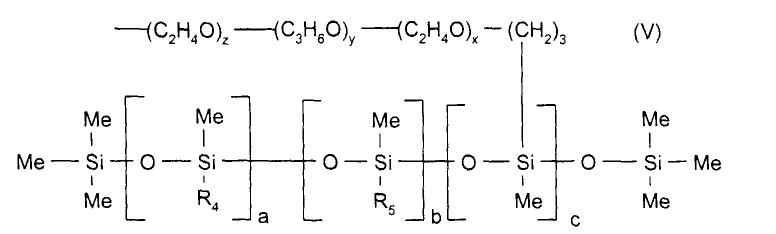
- R₂ est le radical de formule (VI) suivante : 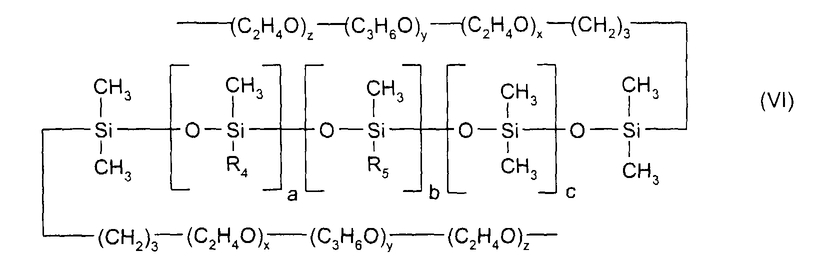 dans lesquelles :
. Me désigne le radical méthyle,
. C₂H₄O représente le groupement -CH₂-CH₂-O-,
. C₃H₆O représente le groupement -CH₂-CH(CH₃)-O-
. a est un nombre entier allant de 0 à 200,
. b est un nombre entier allant de 0 à 200,
. c est un nombre entier allant de 1 à 200,
. R₄ désigne un radical -(CH₂)ₙCH₃ ou phényle, n étant un nombre entier allant de 0 à 10, et de préférence R₄ désigne un radical méthyle
. R₅ est un radical -(CH₂)₃-(OCH₂CH₂)ₓ-(OCH₂CH(CH₃))_{y}-(OCH₂CH₂)_{z}-OH,
. x, y, z sont des nombres entiers, allant indépendamment de 0 à 20, et de préférence x+y+z ≥ 3,
- d et e vont de 1 à 2, avec d+e=3,
- f est égal à 0 ou 1, g est égal à 1 ou 2, avec f+g=2,
- M est choisi dans le groupe constitué de H, Na, K, Li, NH₄ et N(CH₂CH₂OH)₃
- R₃ est choisi parmi : 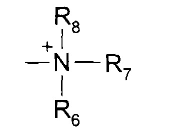 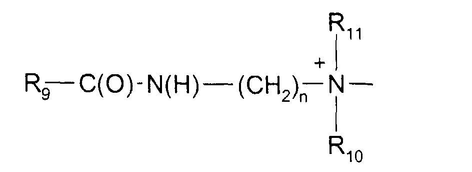 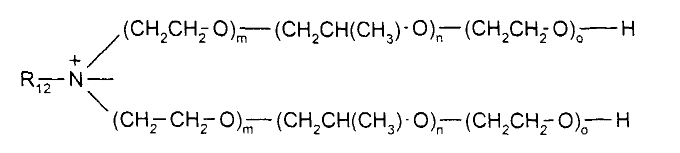 et 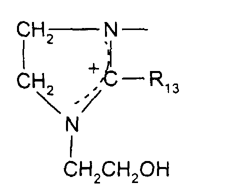 formules dans lesquelles :
. R₆ à R₉ désignent, indépendamment, un radical alkyle ayant 1 à 20 atomes de carbone,
. R₁₀ et R₁₁ désignent, indépendamment, un radical alkyle ayant 1 à 3 atomes de carbone,
. R₁₂ et R₁₃ désignent, indépendamment, un radical alkyle ayant 6 à 20 atomes de carbone,
. m, n et o désignent, indépendamment un nombre entier allant de 0 à 20.

4. Composition selon l'une des revendications 1 à 2, dans laquelle le tensioactif siliconé est choisi parmi les composés de formule (VII) suivante : 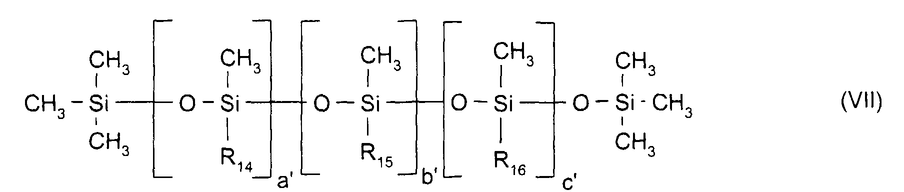 dans laquelle
. R₁₄ désigne un radical alkyle ayant de 1 à 8 atomes de carbone ou un radical phényle,
. R₁₅ désigne un radical
-(CH₂)₃-O-(CH₂CH₂O)ᵤ-(CH₂CH(CH₃)-O)ᵥ-(CH₂CH₂O)_{w}-SO₃⁻M⁺,
M étant choisi parmi Na, K, Li et NH₄,
. R₁₆ désigne un radical
-(CH₂)₃-O-(CH₂CH₂O)ᵤ-(CH₂CH(CH₃)-O)ᵥ-(CH₂CH₂O)_{w}-H,
dans lesquels u, v, w désignent, indépendamment, un nombre entier allant de 0 à 100,
. a' et c' désignent, indépendamment, un nombre entier allant de 0 à 50, et b' désigne un nombre entier allant de 1 à 50.

5. Composition selon l'une des revendications 1 à 2, dans laquelle le tensioactif siliconé est choisi parmi les composés de formule (VIII) suivante : 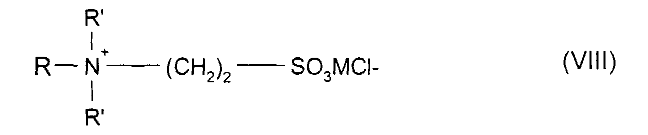 dans laquelle :
. M est choisi dans le groupe constitué de Na, K, Li et NH₄ ,
. R désigne un radical alkyle ayant de 1 à 40 atomes de carbone, et
. R' désigne un radical de formule (V) : 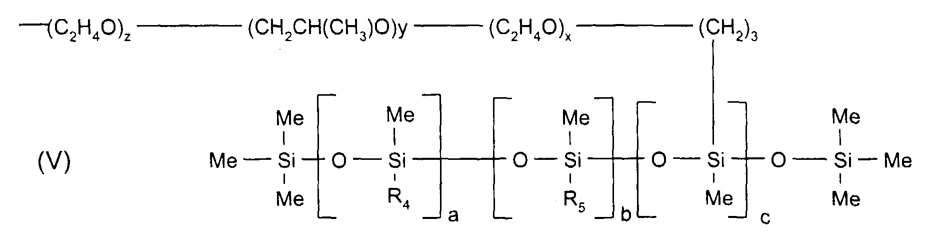 dans laquelle :
. a est un nombre entier allant de 0 à 200,
. b est un nombre entier allant de 0 à 200,
. c est un nombre entier allant de 1 à 200,
. R₄ désigne un radical -(CH₂)ₙCH₃ ou phényle, avec n étant un nombre entier allant de 0 à 10,
. R₅ est un radical -(CH₂)₃-(OCH₂CH₂)ₓ-(OCH₂CH(CH₃))_{y}-(OCH₂CH₂)_{z}-OH, avec x, y et z étant des nombres entiers, allant indépendamment de 0 à 20, et de préférence x+y+z ≥ 3.

6. Composition selon l'une des revendications 1 à 2, dans laquelle le tensioactif siliconé est choisi parmi les composés obtenus par la réaction d'une silicone de formule (IX) : 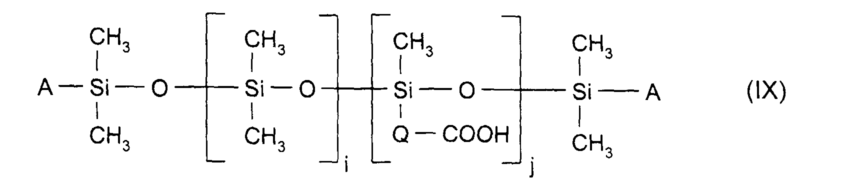 dans laquelle
. Q désigne (CH₂)ᵣ, r étant un nombre entier allant de 3 à 17,
. j désigne soit un nombre entier allant de 1 à 10 et A désigne un radical méthyle, soit j= 0 et A désigne un radical -Q-COOH,
. i désigne un nombre entier allant de 1 à 200,
avec un dérivé de taurine de formule R₁₇-NH-(CH₂)₂-SO₃M, dans laquelle
. R₁₇ désigne un radical alkyle ayant de 1 à 40 atomes de carbone, et
. M est choisi parmi Na, K, Li et NH₄.

7. Composition selon l'une des revendications précédentes, dans laquelle le tensioactif siliconé est présent à une teneur allant de 0,5 à 20% en poids, par rapport au poids total de l'émulsion, de préférence de 2 à 7% en poids.

8. Composition selon l'une des revendications précédentes, dans laquelle l'huile fluorée volatile est choisie parmi, seul ou en mélange, :
a) les perfluorocycloalkyles répondant à la formule (I) suivante : 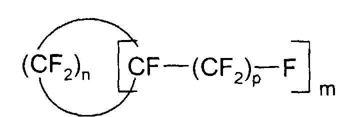 dans laquelle :
- n est 4 ou 5,
- m est 1 ou 2, et
- p est 1, 2 ou 3,
sous réserve que lorsque m=2, les groupements ne sont pas nécessairement en position alpha l'un par rapport à l'autre ;
b) les perfluoroalcanes répondant à la formule (II) suivante:
CF₃-(CF₂)ₘ-CF₂X
dans laquelle :
- m est 2 à 8, et
- X représente Br ou F;
c) les fluoroalkyles ou hétérofluoroalkyles répondant à la formule (III) suivante :
CH₃-(CH₂)ₙ-[Z]ₜ-(CF₂)ₘ-CF₃
dans laquelle :
- t est 0 ou 1,
- n est 0, 1, 2 ou 3,
- m est 2, 3, 4 ou 5, et
- Z est O, S ou NR, R étant un hydrogène, un radical -(CH₂)ₘ-CH₃ ou -(CF₂)ₘ-CF₃,
d) les dérivés de perfluoromorpholine répondant à la formule (IV) suivante : 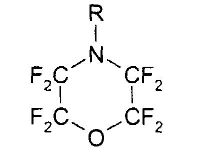 dans laquelle R est un radical perfluoroalkyle en C1-C4.

9. Composition selon l'une des revendications précédentes, dans laquelle l'huile fluorée volatile est choisie parmi, seul ou en mélange, le perfluorométhylcyclopentane, le perfluoro-1,3 diméthylcyclohexane, le dodécafluoropentane, le tetradécafluorohexane, le bromoperfluorooctyle, le nonafluorométhoxybutane, l'éthoxynonafluoroisobutane, la 4-trifluorométhyl perfluoromorpholine.

10. Composition selon l'une des revendications précédentes, dans laquelle l'huile fluorée volatile est présente en une quantité de 0,5 à 50% en poids par rapport au poids de l'émulsion, de préférence en une quantité de 3 à 20% en poids.

11. Composition selon l'une des revendications précédentes, comprenant en outre un corps gras choisi parmi les corps gras siliconés tels que les huiles, les corps gras pâteux, les gommes et les cires de silicone, et/ou les corps gras non siliconés tels que les huiles, les pâteux et les cires végétales, minérales, animales et/ou synthétiques.

12. Composition selon l'une des revendications précédentes, se présentant sous la forme d'une émulsion huile-dans-eau, d'une émulsion eau-dans-huile ou d'une émulsion multiple, de consistance liquide ou semi-liquide du type lait, ou de consistance molle, semi-solide ou solide du type crème, gel voire stick.

13. Composition cosmétique ou pharmaceutique caractérisée par le fait qu'elle comprend, ou est constituée par, une émulsion selon l'une des revendications précédentes.

14. Composition selon la revendication 13, caractérisée par le fait qu'elle se présente sous la forme d'un produit de soin pour le corps, le visage, le cuir chevelu et/ou les cheveux tel qu'une crème, un lait, un gel ou un sérum; d'un produit solaire ou autobronzant; d'un produit de maquillage tel qu'un fond de teint, un fard à joues ou à paupières, un eye-liner, un mascara ou un rouge à lèvres.

15. Utilisation d'un tensioactif siliconé comprenant au moins un groupement anionique pour la préparation d'une émulsion stable comprenant une phase aqueuse et une phase grasse qui comprend au moins une huile volatile fluorée.
